Europäisches Patentamt

European Patent Office

Office européen des brevets

(19)

(11) Publication number: **0 281 246**
**A2**

# EUROPEAN PATENT APPLICATION

(12)

(21) Application number: **88300865.8**

(22) Date of filing: **02.02.88**

(51) Int. Cl.⁴: **C12N 15/00 , C12N 5/00**

---

The title of the invention has been amended (Guidelines for Examination in the EPO, A-III, 7.3).

(30) Priority: **04.02.87 US 10871**

(43) Date of publication of application:
**07.09.88 Bulletin 88/36**

(84) Designated Contracting States:
**AT BE CH DE ES FR GB GR IT LI LU NL SE**

(71) Applicant: **INVITRON CORPORATION**
**4649 Le Bourget Drive**
**St. Louis Missouri 63134(US)**

(72) Inventor: **Simonsen, Christain C.**
**1509 Parkview Avenue**
**San Jose California 95130(US)**

(74) Representative: **Goldin, Douglas Michael et al**
**J.A. KEMP & CO. 14, South Square Gray's Inn**
**London WC1R 5EU(GB)**

---

(54) **Method to enhance amplification and expression of foreign genes.**

(57) A method for increasing production of a heterologous protein employs a strong expression cassette for that protein in combination with a weak expression cassette for an amplifiable gene. Subsequent amplification is effective in attaining high levels of production from the strong cassette in comparison with those obtained when the amplifiable gene is also present in an efficiently designed expression system.

EP 0 281 246 A2

## METHOD TO ENHANCE AMPLIFIED FOREIGN GENE EXPRESSION

Field of the Invention

The invention relates to the production of proteins in eucaryotic cells by recombinant DNA techniques. More specifically, it relates to production of high levels of these proteins through gene amplification induced by coamplification of a drug-resistance gene.

Background of the Invention

In a seminal paper, by Schimke, R.T., et al, Science (1978) 202:1051, the amplification of the gene encoding dihydrofolate reductase (DHFR), which confers resistance to certain antimetabolic drugs such as methotrexate (MTX), was dissolved to coamplify surrounding DNA sequences of considerable length. Advantage was taken of this property in enhancing the level of expression of a foreign gene in mammalian transformants with recombinant DNA by linking the foreign protein encoding sequences to the gene for DHFR and then culturing mammalian cells transformed with this linked sequence in the presence of increasing amounts of methotrexate. Colonies which has acquired resistance to methotrexate survived at higher and higher levels, apparently through amplification of the copy numbers of the DHFR gene and, concomitantly, the linked foreign protein encoding sequence. See, for example, U.S. Patent 4,399,216 to Axel.

The expression of foreign genes in mammalian cell cultures per se has been known for some time. Historically, of course, this has been achieved by utilizing recombinant viruses (see, for example, Mulligan, R.C., et al, Nature (1979) 277:108-114, which describes the synthesis of rabbit beta globin in cultured monkey cells under the control of promoters and other control sequences in the SV40 genome. However, other techniques for transfecting mammalian cells, including calcium phosphate precipitation, DEAE dextran treatment, or microinjection, have permitted the use of DNA per se, not limited to viral vectors. See, for example, Cappecchi, M.R., Cell (1980) 22:479-488. Using these techniques, both viral and mammalian genes, and, indeed, bacterial genes have been expressed (Mulligan, R.C., et al, Science (1980) 209:1422-1427 (E coli xanthine-guanine phosphoribosyl transferase, XGPRT) ; Subramani, S., et al, Mol Cell Biol - (1981) 1:854-864 (mouse dihydrofolate reductase); and Southern, P.J., et al, J Mol Appl Genet (1982) 1:327-341, (bacterial phosphotransferases conferring G418 resistance). The expression of the gene encoding DHFR per se under the control of the adenovirus late promoter in DHFR deficient CHO cells has also been described (Kaufman, R.J., et al, Mol. Cell Biol. (1982) 2:1304-1319). The expression of DHFR in DHFR deficient CHO cells and amplification of this expression, where the expression is under the control of the mouse DHFR gene's own promoter is described by Gasser, C.S., et al, Proc Natl Acad Sci USA (1982) 79:6522-6526.

Specific applications of the use of DHFR amplification to enhance the production of a recombinant protein in DHFR deficient cells has also been described.

For example, EPO Application Publication No. 117,059 published 29 August 1984 describes amplified production of tissue plasminogen activator (tPA) in DHFR deficient host cells transfected with plasmid vector or vectors containing coding sequences for both DHFR and tPA. Each coding sequence is individually linked to a promoter, specifically to the SV40 early promoter in each case. European Application No. 117,060 published on the same day described similar work involving transfection with a plasmid containing the tPA and a mutant DHFR on a plasmid also controlled by the SV40 early promoter. The same vectors are also described in issued UK Patent GB 2119804B. In all of these cases, the DHFR expression behaves as a selectable marker in DHFR-deficient cells; or the mutant DHFR may serve as a selectable marker in cells containing the normal form of DHFR as well. The ability of DHFR to amplify in the presence of methotrexate is used to advantage increasing the amount of protein production by coamplification of this gene.

In all cases known to Applicants involving coamplification and expression of additional DNA sequences, the DNA encoding the desired heterologous protein and the DNA encoding DHFR have bee placed under control of strong promoters, such as SV40 early or late promoters of adenovirus promoters. Because DHFR expression under such promoters is very effective, relatively little gene amplification is needed in order to protect the cells against the drug methotrexate used to stimulate the amplification. By limiting the effectiveness of DHFR production, the expression systems of the present invention result in higher gene amplification than was attainable with the systems already disclosed in the art.

Disclosure of the Invention

The invention provides a system for expressing desired recombinant genes in animal cells, in particular, in DHFR deficient cells, which results in high production levels of the encoded desired protein. The system takes advantage of balancing the production levels of the desired protein and DHFR to encourage the cells harboring the system to amplify both genes to very high levels.

In one aspect, the invention relates to an expression system useful for increasing or controlling the levels of expression of a heterologous, i.e., exogenously introduced, gene in animal cell which has been modified to contain it. The expression system may be maintained on a separate extrachromosomal plasmid, but is more commonly integrated into the host cell chromosome. The system itself is a DNA sequence or a pair of DNA sequences which will result, when integrated into the chromosome in a colinear DNA sequence. It contains the heterologous gene operably and optimally linked to strong control sequences which are operable in the host cell, and also contains within a reasonable distance when colinear, a coding sequence for DHFR or the other amplifiable gene contained in a comparatively weak expression cassette also operable in the same host.

In other aspects, the invention relates to cells which contain this expression system, to methods of producing proteins using these cells, and to the proteins thus produced.

It should be understood that the concept of the invention resides in coamplification of the expression cassette for an amplifiable gene with that for an expression cassette for a desired protein by balancing the relative strengths of the expression cassettes. Thus, the nature of amplifiable gene is not critical to the invention, and DHFR is illustrated herein simply as a convenient example. Other expression cassettes containing genes for appropriate alternate amplifiable gene sequences are by no means excluded.

Brief Description of the Drawings

Figure 1 shows the significant features of the plasmid pSTH-MDH which contains one embodiment of the expression system of the invention.

Figure 2 shows an outline of the construction of intermediate vectors useful in the invention.

Figure 3 shows an outline of the construction of expression vectors for DHFR, tPA and for both.

Modes of Carrying Out the Invention

A. Definitions

As used herein, "amplifiable gene sequence" refers to a DNA encoding a protein which has a metabolic function such that in response to an environmental stimulus the level of protein production is increased, at least in part because of synthesis of multiple gene copies or amplification. DHFR is the best known example, others are, or may become, available.

"Dihydrofolate reductase" or "DHFR" refers either to the protein or the gene encoding it, with or without introns, as will be clear from the context. DHFR protein includes proteins having activity in standard assays for the catalysts of reduction of dihydrofolate and includes both "wild type" and "mutant" forms.

Mutant forms of DHFR are known which have reduced binding capacity for drugs such as methotrexate. These mutants are specifically included within the definition. In general, however, the wild type sequences are more useful for amplification. Because the mutant DHFR is only weakly binding to methotrexate, the cells are inherently resistant to this drug, and amplification is not much encouraged by its presence. On the other hand, the mutant form of DHFR is useful in serving as a selectable marker for transformation into cells which are not DHFR deficient. Even under these circumstances, i.e., the presence of both mutant and nonmutant forms of DHFR in the cell, the gene encoding the nonstrongly binding (mutant) DHFR undergoes some modest amplificiation. However, for use in the systems of the invention, use of wild type DHFR encoding sequences in DHFR-deficient cells is greatly preferred.

It is again stated that although DHFR is a well-known and widely used amplifiable gene system, the invention could employ other amplifiable genes as well, such as adenosine deaminase.

"Expression system" refers to a DNA segment which contains at least one "expression cassette", which cassette contains a coding sequence to be expressed and the control sequence, operably linked to it, which enable its expression. The expression systems of the present invention comprise at least two such expression cassettes--one being a strong expression cassette for the desired protein and the other being a weak expression cassette for the DHFR. Each cassette may be provided on a separate DNA sequence vector or, preferably, on the same vector. The system will be colinear when integrated into the genome.

The terms "strong" and "weak" are, of course, relative, and are further defined below. In order for amplification of the DHFR sequences to involve the other cassette, of course, the two cassettes must be sufficiently close together to behave similarly in response to the amplification stimulus and can either both be integrated on the chromosome of the host cell, or maintained together separate from the chromosome on a plasmid. The distance between the cassettes which is needed is not particularly short, and ultimate placement of the two cassettes within a distance of even 50-200 kb of each other is workable. The orientation of the two cassettes may either be the same or opposite. Of course, the more closely proximal the two cassettes are when transfected, the more likely it is that they will be colinearly integrated into the chromosome or maintained together during replication.

Since amplification spans such a long DNA distance, it is feasible to cotransform the host cells with the expression cassettes on separate vectors, although a single vector is preferred for efficiency. The probability of cointegration on the same chromosome is increased thereby. However, sufficient efficiency is obtained when two vectors are used that this method must be considered within the scope of the invention. Thus, the "expression system" of the invention comprises the two expression cassettes in the same transformation mixture, whether residing on the same plasmid or vector or whether on separate DNA pieces in the mixture.

"Control sequences" refer to DNA segments on the same oligonucleotide molecule as the relevant coding sequence which are necessary and sufficient for expression of the coding sequence, and are part of a particular expression cassette. Typically such control sequences include a promoter, and one or more of transcription terminators, and polyadenylation signals, and may additionally include enhancers, splice signals, and so forth. The critical element with respect to the expression systems of the invention is the relative strength of the expression cassettes, which is a function of the nature of each and all of these features as well as of their placement relative to each other and relative to the coding sequence.

"Heterologous" protein refers to a protein produced by a host cell wherein the gene encoding the protein was introduced exogenously. The protein may, in fact, be identical with protein produced by the host from its own genome. However, as used herein "heterologous" refers to the origin of the gene rather than the nature of the protein, itself. Thus, although a host cell may, for example, produce urokinase as a normal member of its complement of proteins, urokinase may be referred to as a "heterologous" protein herein when, in addition to the "native" material, the host has accepted an expression system which includes a DNA segment encoding urokinase, and is able to effect expression of this exogenously introduced gene.

"Strong" and "weak" expression systems are difficult to quantitate, but it is generally understood that certain control sequences in appropriate configuration with coding segments are better able to effect transcription and subsequent translation into protein than are others. Factors which affect the level of expression include the inherent strength of the promoter sequences; the configuration of these promoter sequences with respect to the start of the message and of the start codon for the coding sequence; the nature of the message with respect to its secondary sequence, which is a reflection of suitable codon choice and possibly of intron sequences; the effectiveness of polyadenylation signals; and so forth. In general, any factors which can be marshaled to strengthen the expression cassette for the production of the desired protein, and any which can be contrived to weaken the cassette designed for the expression of the amplifiable gene, such as DHFR, providing the expression is not so depressed as to be totally ineffective, are within the scope of the invention.

B. General Description

Construction of the expression system of the invention employs standard methods generally known in the art for "cutting and pasting" of DNA segments. In general, a plasmid vector is formed from various pieces containing the control sequence for the two cassettes, two coding sequences, and for convenience, an origin of replication in bacterial cells. Alternatively, each cassette may be placed on a separate vector, prior to mixing for transformation. Of course, viral vectors can be used as well and the two expression cassettes can be placed on a viral genome which has been modified to carry these recombinant DNAs.

The DNA encoding the heterologous protein may be chosen from many known sequences in the art or may encode a protein whose DNA is being cloned for the first time. When the host is chosen so that expression takes place in animal cell systems, the presence of introns does not impede production of the protein so long as the correct splice signals are included. Therefore, either genomic sequences containing introns or cDNA sequences lacking them may be used, or the heterologous gene may be an arbitrary combination of DNAs of various genomic, cDNA, or synthetic origins. Of course, the introns in a genomic sequence may not match precisely the introns as they occur in the native gene and the redundancy of the code permits many variations on the naturally occurring sequence.

The protein encoded by the heterologous gene may be any of those desired to be produced by recombinant techniques, including, but not limited to the tissue plasminogen activator exemplified herein, other enzymes such as urokinase, lung surfactant apoprotein, and protease nexin, immunomodulatory agents such as the interferons, hormones such as vasopressin, human growth hormone, other mammalian/animal growth hormones, chorionic gonadotropin, luteinizing hormone, ACTH, and the like, lymphokines such as IL-1, IL-2 and IL-3, the various colony stimulating factors, various suppressor factors, growth factors such as fibroblast growth factor, epidermal growth factor, nerve growth factor, insulin-like growth factor, and osteogenic growth factors and so forth, antimicrobial peptides, and in short, any protein whose production at high levels is desired.

In some circumstances, the proteins produced may not be intended to be an end-product in themselves, but may aid the host cell in some aspect of its metabolism, or may be required to catalyze another chemical conversion. In particular, these proteins may be enzymes which are responsible for conversions which result in the production of useful non-protein products, such as, for example, steroid dehydrogenases, which convert readily available starting materials into more expensive products. In addition, or in the alternative, the proteins may have a general desirable effect, such as conferring immortality on the cell or increasing the efficiency with which is uses energy sources. Thus, not only genes encoding desired protein products per se, but any exogenously introduced protein-encoding DNA is applicable to the method of the invention.

The invention lies in the appropriate selection and design of the expression cassettes to obtain the most effective relative strengths. Significant factors in this design include the choice of promoters and other control sequences in each expression cassette, in the choice of their disposition relative to the coding sequence of the cassette and to each other, and even the choice of codons in the coding sequences. It is central to the concept of the invention that the cassette for expression of the amplifiable gene sequence be markedly weaker than that for the expression of the heterologous protein-encoding gene.

In order for the system of the invention to be effective, the strength of the cassette for the desired protein should be approximately two times greater than that associated with the amplifiable gene, such as DHFR-encoding, sequence. Of course, this is an arbitrary ratio, and the efficacy of the invention depends on the degree of relative strength. Thus, it is perhaps more accurate to say that it is preferred that the foreign gene expression cassette be at least two times stronger than the amplifiable gene expression cassette, preferably five times stronger, and most preferably ten times stronger. By "stronger" is meant relative ability to produce the relevant protein under identical testing conditions--i.e., more effective in producing protein or effecting expression.

Some standard assays for expression strength in mammalian cells are available and suitable for measurement of certain aspects, such as that utilizing the production of CAT (chloramphenicol acetyl transferase), an easily assessable enzymatic activity. The CAT assay can be used to assess the aspects of the expression cassettes which reside in the choice of control sequences and their relative placement. Of course, it is note useful to assess the effect of codon choice in the genes to be expressed. However, this can be done in a straightforward manner by testing each cassette independently transformed into the host cells for production of either the amplifiable gene product, such as DHFR or the desired protein, as the case may be. Thus, it is a straightforward matter to test whether or not the relative efficiency of expression of the cassette for the production of the desired protein is or is not greater than twofold that of the amplifiable gene product production cassette.

Typically, suitable promoters for driving the production of the heterologous protein are the strong promoters such as the viral promoters; for example, the SV40 early promoter or adenovirus major late promoter in mammalian cells, the Rous sarcoma virus promoter in avian cells, and murine retroviral LTRs (Harvey sarcoma virus). Suitable weak promoters for the DHFR cassette if DHFR is the amplifiable gene include that normally associated with the DHFR sequence from a mammalian species, SV40 late promoter since T-antigen is absent, and most cellular promoters.

The level of the expression of desired protein relative to that of the amplifiable gene product could also be enhanced by manipulation of the transcription termination signals by the placement of the sequence in better or worse position with respect to the colinearly disposed controls, and by appropriate choice of codons to confer secondary structure on the messenger RNA encoding the desired protein which is more stable than that of the secondary structure of the mRNA encoding for example, DHFR. All of these approaches capitalize on factors affecting the power of expression for a gene encoding a particular protein, and design of this disparity between expression cassettes in a multitude of approaches is understood by those in the art.

The construction of the expression system is designed according to the origin of the segments of DNA employed, and for convenience uses an origin of replication replicable in bacterial systems, for example an E. coli origin of replication such as that found in the pBR322 series of plasmids or in bacteriophage. The expression system, once constructed, can then be conveniently amplified in a procaryotic system using transformation and cultivation methods appropriate to the host as is understood in the art, and the plasmid or phage DNA is isolated for use in the cultured animal cells.

The expression system of this invention is designed for production of desired proteins by recombinant means. Since the production of these proteins in cells with generally similar characteristics to the cell of origin of the protein assures posttranslational modifications which are useful in producing the protein in its most effective form, animal cells which are similar to those in which the protein is normally found are favored hosts. Since many proteins of interest are human or other mammalian proteins, expression of genes encoding them in mammalian cells is highly favored. Other cell types may be more advantageous for nonmammalian proteins--e.g., cells of avian origin for avian protein.

The animal cells appropriate for use in the invention can be any cells which are dependent on the amplifiable gene product, such as DHFR, for DNA synthesis and for growth. Particularly advantageous for this invention are cells deficient in their own DHFR protein, since these cells are most dependent on the DHFR produced by the expression system of the invention. However, cells which natively produce DHFR may also be employed, since the heterologous sequences provided by the expression system will also be marshaled to overcome the effects of methotrexate. While mammalian cells are of greatest interest due to the importance of the proteins they ordinarily produce, other animal cells are also usable in the invention. Particularly preferred are vertebrate cells, including those derived from avian species. Mammalian cells are, however, the most preferred.

The cells are transformed or transfected according to standard methods such as the calcium phosphate precipitation method of Graham and Van der Eb in its various modifications, microinjection, electroporation, protoplast fusion, and the like.

The transfection mixture may contain the expression cassettes on a single plasmid or vector or may include a mixture of separate vectors containing the cassettes. While it is not required to do so, efficiency of transfection and chromosome integration is generally improved by first linearalizing any plasmid vectors, and further improved by treating them with suitable restriction enzymes so as to delete the bacterial sequences which has been used for amplification. Thus, in a particularly preferred approach, a plasmid vector is first linearalized and treated with enzymes which delete the bacterial sequences and the desired expression cassette(s) purified away for transfection; this latter especially encourages recombination with the chromosomal DNA. The coamplification will be effective if the two cassettes are integrated onto the same chromosome within a distance of 50-200 kb; therefore, the distance of their separation on a single vector utilized to transfect the host is virtually irrelevant. As is mentioned above, separate expression cassettes can also be used.

The cells containing the expression system are then cultured under conditions suitable for the host and for amplification of the amplifiable gene. These conditions depend, of course, on the nature and nutritional requirements of the host, on the nature of the marker for selection used, and on the nature of the amplifiable gene.

For example, in one embodiment, both the marker and amplifiable gene may be DHFR. In this case, transfection is done into DHFR-deficient cells, which are then cultured in media which will result in DHFR amplification. The typical medium contains small amounts of methotrexate, a drug highly deleterious to the cells. However, the effects of the drug are overcome by an excess of DHFR protein. Generally the selection of methotrexate resistant colonies is obtained by picking individual colonies or pools which survive increasing concentrations of the drug; such methods for selecting individual cultures with high methotrexate resistance are well known in the art. In general, the initial concentrations of methotrexate are in the nM range, and cells which contain sufficiently amplified sequences to survive in the $\mu$M range can eventually be obtained. However, maintaining the high levels of methotrexate which are ordinarily necessary to maintain gene amplification is generally undesirable for heterologous protein production, since the compensation for this drug throws the cellular metabolism out of balance. Cells can be assessed for their ability to produce the heterologous protein along with the increasing selection pressure for their resistance to methotrexate, and a level of methotrexate commensurate with optimum protein production can then be employed for culturing the cells to produce the desired protein.

Of course, when DHFR is used as the amplifiable gene, drugs other than methotrexate which exert the same effect on metabolism could also be used. However, methotrexate is readily available and convenient.

Once the optimum level of methotrexate has been determined, as judged by the optimum level of heterologous protein production, the cells are cultured specifically for protein production in media containing the ascertained level of methotrexate. For production of the heterologous protein, the cells are grown to logarithmic phase by passaging several times in the medium. If production of encoded protein product is the desired result, the protein produced is isolated directly from the culture (or lysed cells, depending on whether the foreign sequence contains an operable signal to effect secretion). If the protein is produced in order to alter the cells metabolism, of course, no isolation is needed or desired.

In the alternative, the cells are grown up and then placed in a static maintenance reactor (SMR) culture system which maintains nonproliferating cells for extended time periods permitting them to continue production of the desired protein, but without requiring added metabolites for growth. Such reactor systems are described, for instance, in US 4,537,860, incorporated herein by reference.

## Examples

The following examples are intended to illustrate but not to limit the invention.

## Example 1

### Construction of Plasmids Containing the Expression Cassettes

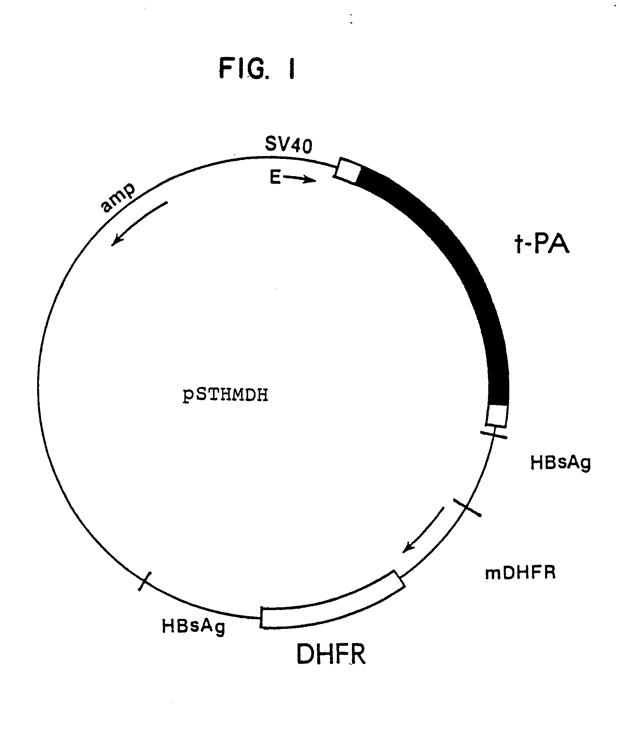

The plasmid pSTH-MDH, whose features are shown in Figure 1, is a typical embodiment of the expression system of the invention and contains the cDNA encoding tPA under the control of the SV40 early promoter and is followed by a polyadenylation signal derived from hepatitis B virus. It also contains the gene for murine DHFR, under the control of its own promoter, and also followed by a polyadenylation signal derived from HBV. The plasmid further contains the E coli origin of replication derived from the pML plasmid, along with the ampicillin resistance gene.

Plasmid pSTH-MDH thus exemplifies the expression system of the invention residing on a single plasmid. Additional vectors were constructed wherein the two expression cassettes reside on separate vectors. The construction of each of these plasmids is described below.

### Construction of Intermediates

Several intermediate vectors containing the various elements of the invention expression cassettes and useful in the construction of them in expression vectors were prepared, as follows:

pSVoriHBV3' contains the origin of replication and early and late promoters of SV40 upstream of the 3' termination sequences from the hepatitis B surface antigen gene with insertion sites for a foreign gene between them. pSVoriHBV3' is constructed from pML, SV40, and HBV. pML is digested with EcoRI, blunted with Klenow, and then digested with HindIII. The vector fragment containing the E. coli origin of replication and the ampicillin resistance gene is isolated and ligated to the isolated 540 bp fragment containing the early and late promoters and origin of replication of SV40, obtained by digestion of SV40 DNA by HindIII and HincII. The resulting vector, designated pSVori, is then digested with BamHI for acceptance of a 585 bp fragment isolated from a BamHI/BglII digest of HBV DNA which contains the 3' termination sequences of the surface antigen gene. Correct orientation is confirmed by restriction analysis - digestion with HindIII and BamHI yields a 350 bp fragment from the correct vector. The resulting ligated vector, pSVoriHBV3', thus contains the SV40 promoter and origin sequences upstream of the HBV terminator and permits a coding sequence to be inserted conveniently between them.

Also prepared was ptPA-BAL17, which contains the tailored upstream portion of the tPA gene in a bacterial replication vector. The tPA cDNA is furnished by the vector pMON-1068, which is a bacterial vector containing an insert of the entire cDNA sequence obtained for tPA as described in Pennica, D. et al, Nature (1983) 301:214-221. Of course, any bacterial replication vector containing this coding sequence could just as well have been used, and the restriction sites designated below fall within the disclosed sequence of the tPA cDNA set forth in the Nature reference. pMON-1068 is first digested with BamHI to excise the tPA encoding cDNA and then with BAL-31 to chew back at each end of the gene. Digestion with BAL-31 was continued until analysis of the lengths and sequence of linear fragments indicated that the 5' end of the fragment was within 17 bp of the ATG start codon. The precise distance of chew-back is not critical so long as it is within sufficiently short distance to permit the ATG to be placed an operable distance from the promoter in the expression cassette. A separation in this fragment of the 5' terminus from the ATG of about 10 bp is, in fact, preferred. The selected linear fragment was then digested with SacI, which cuts inside the coding sequence of the tPA gene, and the resulting blunt/SacI fragment was isolated. This contains the suitably tailored 5' end of the gene and was ligated into SacI/HincII-digested pUC13 to give the intermediate plasmid ptPA-BAL17.

pUC-DHFR was used as a cloning vector for the DHFR-encoding sequences, absent their associated control sequences. pUC-DHFR was constructed by digesting pDHFR-11 (Simonsen, C.C., et al, Proc Natl Acad Sci USA (1983) 80:2495-2499) with Fnu4HI, blunting with Klenow and then digesting with BglII to isolate the 660 bp fragment as there described, and ligating this fragment into pUC13 which had been digested with HincII and BamHI. Thus, pUC-DHFR represents a straightforward cloning vector for DHFR analogous to the ptPA-BAL17 vector described for the 5' portion of the tPA gene above.

Finally, a separate cloning vector for the termination sequences derived from the hepatitis B surface antigen gene, pUC-HBV3', was constructed by digesting HBV DNA with BamHI and BglII and isolating the 585 bp fragment, as described above, and ligating this fragment into BamHI-digested pUC13.

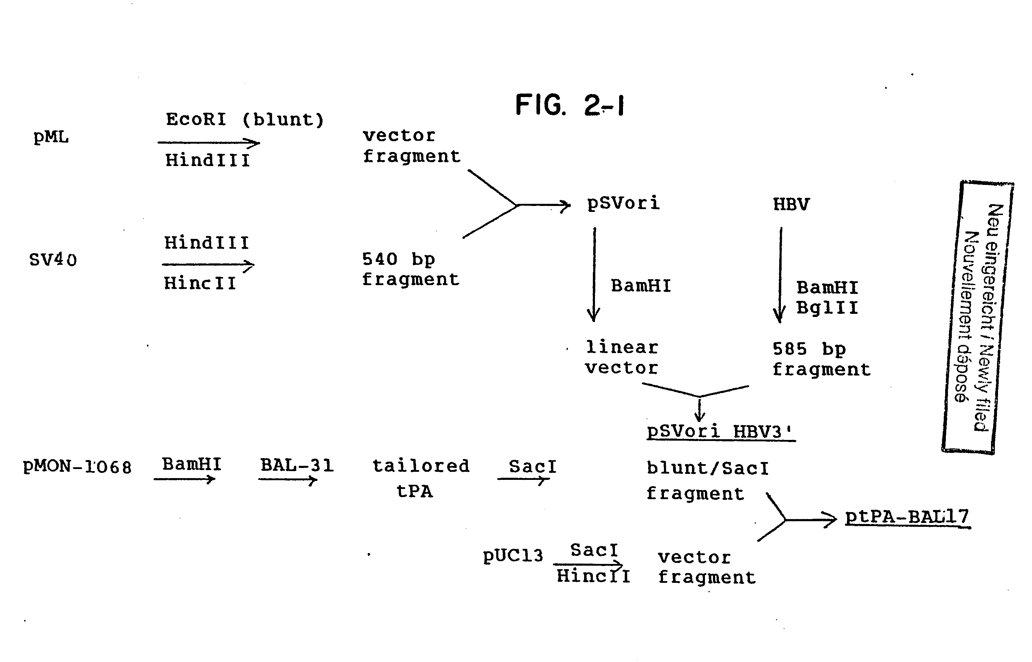

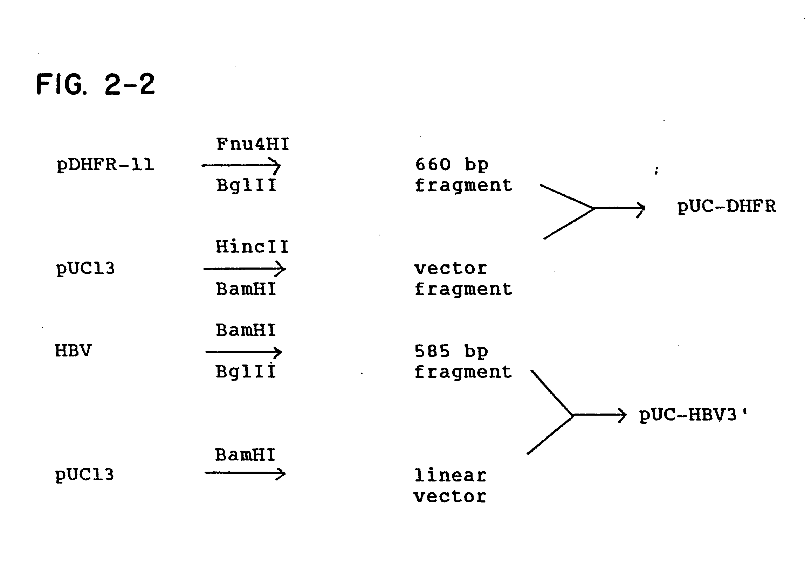

The construction of the foregoing vector intermediate is outlined in Figure 2.

## Construction of Expression Vectors

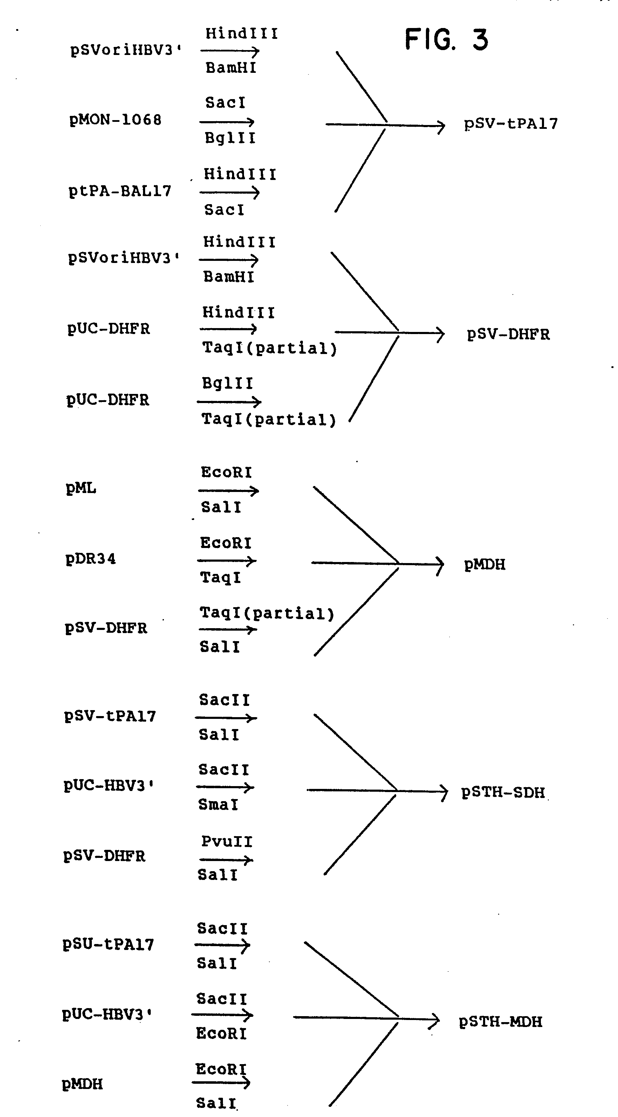

The above-described intermediate vectors were used, along with additional components available in the art, to construct expression cassettes contained in plasmid vectors as outlined in Figure 3.

pSV-tPA17, which contains the full-length tPA coding sequence under control of SV40 promoter and HBV terminating sequences was prepared as a three-way ligation of the vector fragment from pSVoriHBV3' digested with HindIII and BamHI, which thus provides the promoter and terminator along with vector sequences; the 3' of tPA obtained by SacI/BglII digestion of pMON-1068; and the tailored 5' portion of the tPA coding sequence, which was obtained as a HindIII/SacI digest of ptPA-BAL17. The resulting ligation mixture was transfected into E. coli, the transformants selected for ampicillin resistance, and plasmid DNA containing the desired pSV-tPA17 isolated.

The counterpart vector for DHFR expression, designated pSV-DHFR, was also obtained in a three-way ligation. Again the vector fragment obtained from HindIII/BamHI digestion of pSVoriHBV3' was used to provide the control sequences, and the 5' and 3' portions of the DHFR coding sequence were obtained by digestion of pUC-DHFR with HindIII and TaqI (partial) and with BglII and TaqI (partial), respectively. The ligation mixture was used to transform E. coli, ampicillin resistant transformants were selected, and plasmid DNA, designated pSV-DHFR was isolated.

The two foregoing expression vectors, of course, contain expression cassettes of comparable strength for the tPA and DHFR DNA sequences. These expression systems were combined on the same plasmid, pSTH-SDH, by a three-way ligation of the appropriate fragments obtained by SacII/SalI digestion of pSV-tPA17, SacII/SmaI digestion of pUC-HBV3', and the PvuII/SalI fragment of pSV-DHFR. The ligation mixture was treated as above and the desired plasmid, pSTH-SDH was isolated.

A single plasmid containing a weak expression system for the DHFR coding sequence was also prepared. This plasmid, pMDH, was obtained in a 3-way ligation using the 1 kb fragment obtained by EcoRI/TaqI (partial) digestion of pDR34, the vector fragment from EcoRI/SalI-digested pML, and the 3' end of the gene isolated from TaqI (partial)/SalI digested pSV-DHFR. (The pDR34 vector is described by Gasser, C.S., et al, Proc Natl Acad Sci USA (1982) 79:6522-6526, supra) and contains the mouse DHFR gene linked to its own promoter.) The resulting vector, pMDH, is analogous to pSV-DHFR, except that the DHFR gene is under control of the murine DHFR promoter. The weak expression cassette residing on pMDH and strong expression cassette residing on pSV-tPA17, when used in admixture to transfect suitable DHFR-deficient cells, thus constitute one embodiment of the expression system of the invention.

Finally, pSTH-MDH, which contains the expression system of the invention on a single vector, was constructed as a three-way ligation of the appropriate isolated fragments of pSV-tPA17, pMDH, and pUC-

HBV3'. pSV-tPA17 is digested with SacII and SalI, pMDH with EcoRI and SmaI, and pUC-HBV3' with SacII and EcoRI. This ligation results in the construction shown in Figure 1, wherein the same vector contains two expression cassettes, a strong cassette for the desired tPA sequences and a weaker cassette for the amplifiable DHFR gene.

Example 2

The expression vectors of Example 1 were used to transform DHFR deficient Chinese hamster ovary cells using the procedure of Graham and Van der Eb as modified by Wigler, M., et al, Proc Natl Acad Sci USA (1980) 77:3567-3570. The host cell line is readily available to the public and is described in Urlaub, G. and Chasin, L., Proc Natl Acad Sci USA (1980) 77:4216-4220.

Successful transformants were selected and then amplified in media containing increasing levels of methotrexate beginning at 10 nM. Amplification of the DHFR gene and further selection was obtained at increasing levels of methotrexate up to 10 μM. Recombinant tPA production was also monitored in cell lysates using the commercially available ELISA method distributed by American Diagnostics, Greenwich, CN.

Table 1 shows the results as an average of several experiments of transformation efficiency and tPA production at various levels of methotrexate (MTX) for various plasmids used in the transformation mixture.

## Table 1

| Plasmid(s) | #col/$10^6$ | tPA/cell(pg) 10nM MTX | tPA/cell(pg) 250nM MTX | tPA/cell(pg) 10μM MTX |
|---|---|---|---|---|
| pSV-DHFR | 500 | 0.0 | 0.0 | 0.0 |
| pMDH | 25 | 0.0 | 0.0 | 0.0 |
| pSV-tPA17 | 0 | – | – | – |
| pSTH-MDH | 25 | 0.1 | 20.0 | 2.0 |
| pSTH-SDH | 500 | 0.01 | 0.25 | 0.9 |
| pSV-tPA17 + pSV-DHFR | 500 | 0.01 | 0.1 | 0.8 |
| pSV-tPA17 + pMDH | 25 | 0.1 | 5.0 | 1.0 |

As shown in Table 1, because of the selection system employed, no results can be obtained when pSV-tPA is used alone as a transforming vector. The greatest efficiency of transformation is found when DHFR is expressed in the context of efficient expression cassettes, as evidenced by the relatively high number of colonies per $10^6$ cells transformed where pSV-DHFR alone or in combination or pSTH-SDH is used. However, when these systems were cotransfected with tPA expression systems, either on the same vector (pSTH-SDH) or on a mixture of vectors (pSV-tPA plus pSV-DHFR), the levels of tPA produced are quite low at all methotrexate levels tested. On the other hand, while the transformation frequency was less for the vectors and mixtures thereof containing the weak MDH cassette, levels of tPA production were consistently higher, regardless of the position of the tPA cassette on the same vector (pSTH-MDH) or on a different vector (pSV-tPA plus pMDH).

The tPA production level in picograms per cell seemed to reach an optimum value at levels of 250 nM methotrexate, presumably because the cells are healthier at these levels than they are at 10 μM methotrexate (compare columns 3 and 4). The production levels shown (5 - 20 pg per cell) represent, under the culture conditions used, 20-40 μg/ml of cell culture.

A representative cell line obtained from the transfection with, and amplification of, pSTH-MDH have short doubling times relative to the cells obtained when plasmids pSV-tPA17 and pSV-DHFR utilized. One line transformed with pSTH-MDH was designated IBD1, and used to produce tPA in a perfusion reactor. High levels of tPA production were obtained.

## Claims

1. An expression system useful for expression of a heterologous gene in an animal host cell transfected with said system, wherein said system comprises two expression cassettes wherein:
   a first expression cassette consists of said heterologous gene operably linked to control sequences compatible with said host, and
   a second expression cassette consists of a coding sequence for an amplifiable gene operably linked to control sequences compatible with said host,
   wherein the first cassette is at least twofold more effective in effecting expression in said host than the second cassette.

2. The system of claim 1 wherein the first cassette is at least fivefold more effective in effecting expression than the second cassette.

3. The system of claim 1 wherein the first cassette is at least tenfold more effective in effecting expression than the second cassette.

4. The system of any one of the preceding claims wherein the first and second cassettes are colinear.

5. The system of any one of the preceding claims wherein the amplifiable gene is DHFR.

6. The system of any one of the preceding claims wherein the control sequences are compatible with vertebrate cells.

7. The system of claim 6 wherein the control sequences are compatible with mammalian cells.

8. The expression system of any one of the preceding claims wherein the first cassette employs a promoter selected from the SV40 early promoter and the adenovirus major late promoter.

9. The expression system of any one of the preceding claims wherein the amplifiable gene is DHFR and the second cassette employs a promoter which is the native DHFR promoter.

10. The expression system of any one of the preceding claims wherein the heterologous gene encodes tissue plasminogen activator.

11. Host animal cells transfected with the expression system of any one of the preceding claims.

12. Cells according to claim 11 wherein the amplifiable gene is DHFR and which are transfected forms of cells deficient in DHFR.

13. Cells according to claim 11 or 12 which are mammalian cells.

14. Cells according to claim 13 which are Chinese hamster ovary cells.

15. A method of enhancing expression levels for a heterologous gene in an animal host cell which comprises culturing cells according to any one of claims 11 to 14 wherein the amplifiable gene is DHFR in the presence of an effective level of methotrexate.

16. A method according to claim 15 wherein the methotrexate level is in the range 5 nM-500 nM.

# FIG. 1

# FIG. 2-1

pML $\xrightarrow[\text{HindIII}]{\text{EcoRI (blunt)}}$ vector fragment

SV40 $\xrightarrow[\text{HincII}]{\text{HindIII}}$ 540 bp fragment

pSVori    HBV

pSVori $\downarrow$ BamHI

HBV $\downarrow$ BamHI BglII

linear vector

585 bp fragment

pSVori HBV3'

pMON-1068 $\xrightarrow{\text{BamHI}}$ $\xrightarrow{\text{BAL-31}}$ tailored tPA $\xrightarrow{\text{SacI}}$ blunt/SacI fragment

ptPA-BAL17

pUC13 $\xrightarrow[\text{HincII}]{\text{SacI}}$ vector fragment

# FIG. 2-2

pDHFR-11 $\xrightarrow[\text{BglII}]{\text{Fnu4HI}}$ 660 bp fragment

pUC13 $\xrightarrow[\text{BamHI}]{\text{HincII}}$ vector fragment

pUC-DHFR

HBV $\xrightarrow[\text{BglII}]{\text{BamHI}}$ 585 bp fragment

pUC13 $\xrightarrow{\text{BamHI}}$ linear vector

pUC-HBV3'

0 281 246

FIG. 3

pSVoriHBV3' $\xrightarrow[\text{BamHI}]{\text{HindIII}}$

pMON-1068 $\xrightarrow[\text{BglII}]{\text{SacI}}$ → pSV-tPA17

ptPA-BAL17 $\xrightarrow[\text{SacI}]{\text{HindIII}}$

pSVoriHBV3' $\xrightarrow[\text{BamHI}]{\text{HindIII}}$

pUC-DHFR $\xrightarrow[\text{TaqI(partial)}]{\text{HindIII}}$ → pSV-DHFR

pUC-DHFR $\xrightarrow[\text{TaqI(partial)}]{\text{BglII}}$

pML $\xrightarrow[\text{SalI}]{\text{EcoRI}}$

pDR34 $\xrightarrow[\text{TaqI}]{\text{EcoRI}}$ → pMDH

pSV-DHFR $\xrightarrow[\text{SalI}]{\text{TaqI(partial)}}$

pSV-tPA17 $\xrightarrow[\text{SalI}]{\text{SacII}}$

pUC-HBV3' $\xrightarrow[\text{SmaI}]{\text{SacII}}$ → pSTH-SDH

pSV-DHFR $\xrightarrow[\text{SalI}]{\text{PvuII}}$

pSU-tPA17 $\xrightarrow[\text{SalI}]{\text{SacII}}$

pUC-HBV3' $\xrightarrow[\text{EcoRI}]{\text{SacII}}$ → pSTH-MDH

pMDH $\xrightarrow[\text{SalI}]{\text{EcoRI}}$